# EUROPÄISCHE PATENTSCHRIFT

(11) **EP 1 524 201 B1**
(45) Veröffentlichungstag und Bekanntmachung des Hinweises auf die Patenterteilung: **22.05.2013**
(21) Anmeldenummer: 04021119.5
(22) Anmeldetag: 06.09.2004
(51) Int. Cl.: B65D 77/20, B65D 65/46, B65D 85/00, A61M 37/00

(54) **Steriler Farbbehälter für den Einmalgebrauch im Tattoo- und Permanent-Make-up-Bereich**
Disposable, sterile container for colour used in tattooing
Récipient stérile à usage unique, pour couleur employés dans les traitements de tattouages

(30) Priorität: 04.09.2003 DE 10341227
(43) Veröffentlichungstag der Anmeldung: 20.04.2005
(73) Patentinhaber: Junk, Paul, 6462 AB Kerkrade (NL)
(72) Erfinder: Junk, Paul, 6462 AB Kerkrade (NL)
(74) Vertreter: Jostarndt, Hans-Dieter

(56) Entgegenhaltungen:
- DE-U1- 29 916 971
- US-A- 5 833 649

## Beschreibung

Die Erfindung betrifft einen Farbbehälter für Tattoo- und Permanent-Make-up (PMU)-Farben zur Gewährleistung der Sterilität bei der Kundenbehandlung und zur rationellen Verwendung von Tattoo- und PMU-Farbe.

Die Erfindung betrifft ferner ein Verfahren zum Aufbringen von Tattoos oder PMU auf einen menschlichen oder tierischen Körper mit Hilfe von Nadeln unter Verwendung des Farbbehälters.

Für das Tätowieren und Pigmentieren von Farbe in oder unter die Haut wird die einzubringende Farbe meist unmittelbar vor dem Tätowieren oder auch während des Tätowierens aus Farbvorratsbehältern in kleinere Gebrauchbehälter abgefüllt. Zum Tätowieren werden die Nadeln abwechselnd in die mit Farbe gefüllten Gebrauchsbehälter und in die Haut eingetaucht.

Die Verwendung derartiger Gebrauchsbehälter birgt erhebliche Gesundheitsrisiken:
Beim Tätowieren tritt Blut und Serum aus der geöffneten Haut an Nadeln und Farbe. Es besteht ein offener Weg für Keime, Bakterien und Viren zwischen Haut, Nadeln, Farbtopf und Vorratsflasche.

Um die relativ dickflüssige Farbe in die Gebrauchsbehälter abzufüllen, sind die Farbvorratsflaschen meistens mit Pipetten oder Spritzverschlüssen ausgelegt. Ein Kontakt der Vorratsflasche mit dem Gebrauchsbehälter ist nicht ausgeschlossen. Keime und Bakterien können sich in und an den Vorratsflaschen sowie auch in den Gebrauchsbehältern bilden und weiter verbreiten, insbesondere wenn Farbe während einer Tätowierung nachgefüllt wird.

Eine Sterilität der Farbe, Vorratsflaschen und Gebrauchsbehälter ist nicht gewährleistet. Es besteht eine große Gefahr einer Übertragung von Krankheiten wie Hepatitis oder Aids.

Aus der deutschen Gebrauchsmusterschrift DE 299 16 97 U1 ist ein Farbbehälter für Tattoo- oder PMU-Farben bekannt, der aus einem Behälterkörper und einem an diesem anschließenden Pipettierhals besteht, welcher durch einen Einmalverschluss verschlossen ist. Der Behälter kann durch Zusammendrücken auf die Haut entleert werden, dient aber insbesondere als Farbkartusche, die auf einen entsprechenden Filzstutzen eines Tätowier- oder PMU-Stiftes gesteckt wird. Der Einmalverschluss wird entweder manuell geöffnet oder im Falle einer Verwendung als Kartusche im Füllstutzen durchstochen.

Bei dem bekannten Einwegbehälter für Tattoo- oder PMU-Farbe besteht der Nachteil, dass der beim manuellen Öffnen mit unsterilen Händen/Handschuhen kontaminiert werden könnte.

Ein Einsatz als Farbkartusche ist nur bei speziellen, sehr aufwendigen Tätowiergeräten möglich, mit denen zudem kein so individueller Farbeintrag wie durch das abwechselnde Eintauchen der Nadeln in Farbe und Haut erzielt werden kann.

Darüber hinaus gehen aus der US-Patentschrift 5 833 649 ein Verfahren und ein Kit zum Entfernen von Tattoos. Das Kit umfasst dabei Tattoo-Farbe und Farbpigmente, wobei die Tattoo-Farbe mittels der Farbpigmente an die Hautfarbe einer Person angepasst wird. Durch das "Übertätowieren" eines Tattoos mit der Hautfarbe wird das Tattoo dabei entfernt.

Der Erfindung liegt daher die Aufgabe zugrunde, ein Behältnis zu schaffen, das möglichst einfach geöffnet werden kann und das mit allen gängigen Tätowier- und PMU-Stiften verwendet werden kann.

Ferner ist es Aufgabe der Erfindung, ein Verfahren zum Aufbringen von Tattoos oder PMU anzugeben, bei dem die Sterilität in hohem Maße gewährleistet ist und das mit allen gängigen Tätowier- und PMU-Stiften durchgeführt werden kann.

Hinsichtlich des Behältnisses wird die Aufgabe durch einen Farbbehälter für Tattoo- und PMU-Farben mit den Merkmalen des Anspruchs 1 gelöst.

Hinsichtlich des Verfahrens wird die Aufgabe durch ein Verfahren mit den Merkmalen des Anspruchs 9 gelöst.

Insbesondere ist ein Farbbehälter für Tattoo- und PMU-Farben vorgesehen , der aus einem Topf besteht, welcher eine für mindestens eine, vorzugsweise genau eine Tattoo- oder PMU-Behandlung erforderliche Menge an Tattoo- oder PMU-Farbe enthält und mit einer von Nadeln eines Tätowier- oder PMU-Stiftes durchdringbaren Folie dicht verschlossen ist.

Es ist zweckmäßig, für verschiedene Tätowiervorgänge unterschiedlich große Farbbehälter bereitzustellen. Beispielsweise sind für die Tätowierung eines etwa handflächengroßen Tattoos nur etwa 0,5 ml bis 2 ml erforderlich, während für eine Ganzkörpertätowierung im Stil eines Maori-Kriegers wesentlich größere Farbmengen, beispielsweise 2 ml bis 10 ml benötigt werden. Beispielsweise hängt die Farbmenge auch von den Anteilen der jeweiligen Farben in dem beabsichtigten Tattoo ab.

Für vorgegebene Farbkombinationen ist es zweckmäßig, mehrere Farbbehälter mit verschiedenen Farben über die Folie miteinander zu verbinden.

Bei dem erfindungsgemäßen Einweg-Farbbehälter ist das Risiko einer Krankheitsübertragung auf einen Kunden minimiert, und für die Verwendung des Farbbehälters bedarf es keines speziellen Tattoo- oder PMU-Stiftes.

Es ist besonders vorteilhaft, die Form des in dem erfindungsgemäßen Farbbehälter enthaltenen Topfes so auszubilden, dass er einen Zylinder mit Ausnahme einer Grundfläche formbündig umschließt.

In dieser Ausführungsform kann der Farbbehälter mit der geschlossenen Grundfläche stabil auf eine Unterlage gestellt werden oder in einem Ständer mit einer oder mehreren zylinderförmigen Vertiefungen hineingestellt werden.

In einer ebenfalls besonders bevorzugten Ausgestaltung des Farbbehälters bestehen der Topf und die ihn verschließende Folie aus einem bioverträglichen Material.

Darüber hinaus kann der Farbbehälter in einer geeigneten Umverpackung steril verpackt sein und bis zur Benutzung in der Umverpackung steril aufbewahrt werden.

Es ist ebenfalls denkbar, das Sterilisieren nach dem Verpacken durchzuführen. Dies kann etwa durch eine Behandlung mit γ-Strahlung erfolgen.

Bei dem Verfahren zum Aufbringen von Tattoos oder Permanent-Make-up auf einen menschlichen oder tierischen Körper mit Hilfe von Nadeln ist es insbesondere vorgesehen, dass eine einen Farbbehälter verschließende Folie mit den Nadeln durchstochen wird, die Nadeln in eine in dem Farbbehälter befindliche Farbe eingetaucht werden und die Nadeln nachfolgend in die Haut des Körpers hineingestochen werden.

Das erfindungsgemäße Verfahren hat den Vorteil, dass der Farbvorratsbehälter nicht mit den Händen geöffnet werden muss. Durch Öffnen mit den Händen könnte der Behälter und die enthaltene Farbe kontaminiert werden.

Alle unnötigen Kontaktwege werden bei dem erfindungsgemäßen Verfahren vermieden.

Eine vorteilhafte Ausführungsform des erfindungsgemäßen Farbbehälters und eine Veranschaulichung des erfindungsgemäßen Verfahrens werden nachfolgend anhand der Zeichnungen dargestellt.

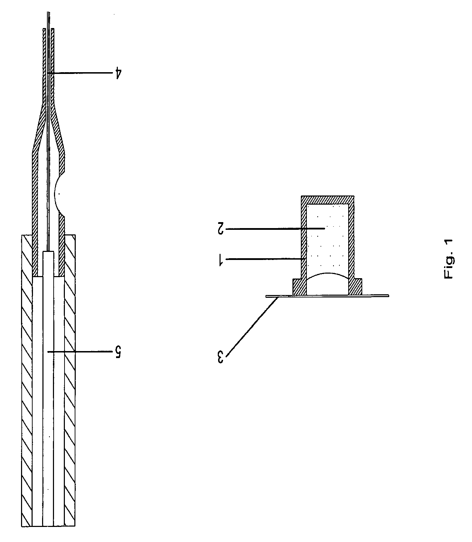

Es zeigt
- FIG. 1 ...: einen Schnitt, der durch einen Farbbehälter für Tattoo- und PMU-Farbe und einen Schnitt durch einen Tattoo- oder PMU-Stift und
- FIG. 2 ...: einen Schnitt, der durch einen Farbbehälter mit durchstoßener Folie und einen in den Farbbehälter eingetauchten Tattoo- oder PMU-Stift.

Der Farbbehälter für Tattoo- und PMU-Farben besteht aus einem zylindrischen Topf (1), der vorzugsweise aus einem bioverträglichen Kunststoff besteht. Der Topf enthält eine für mindestens eine, vorzugsweise genau eine Tattoo- oder PMU-Behandlung ausreichende Menge an Tattoo- oder PMU-Farbe (2) und ist mit einer Folie (3) verschlossen, die mit dem Topf verschweißt oder verklebt ist und die vorzugsweise ebenfalls aus einem bioverträglichen Kunststoff besteht.

Der Farbbehälter besitzt typischerweise ein Füllvolumen von 0,5 ml bis 10 ml und weist die entsprechenden Abmessungen auf.

Vorzugsweise wird der Topf (1) nicht bis zum oberen Rand mit der Farbe (2) befüllt, um ein Herausspritzen der Farbe (2) aus dem Topf beim Einstechen der Nadeln (4) und ein Überlaufen des Topfes (1) beim Eintauchen der Nadeln (4) zu verhindern.

Der zu der offenen Seite hin gerichtete Abschluss des Topfes ist als Wulst ausgebildet, um eine größere Auflagefläche für die Folie (3) zu schaffen.

Der Farbbehälter wird unter sterilen Bedingungen mit der Farbe (2) befüllt und mit der Folie (3) verschlossen. Er kann nachfolgend und ebenfalls unter sterilen Bedingungen in eine Umverpackung verpackt werden und so verkauft, verschickt und steril aufbewahrt werden.

Ein nach dem Verpacken erfolgendes Sterilisieren durch eine Behandlung mit γ-Strahlung kann ebenfalls vorgesehen sein.

Das Material und die Dicke der Folie (3) sind so gestaltet, dass die Folie (3) mit den Nadeln (4) eines Tätowier- oder PMU-Stiftes (5) durchstoßen werden kann.

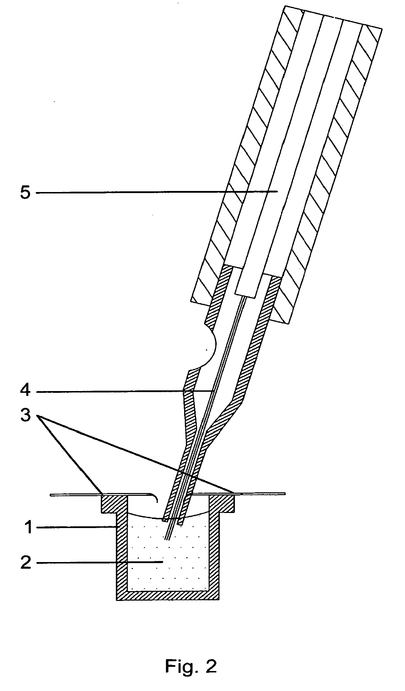

Beim Tätowieren der Haut eines Kunden oder beim Aufbringen von Permanent-Make-up wird die den Farbtopf (1) verschließende Folie (3) mit den Nadeln (4) eines Tätowier- oder PMU-Stiftes durchstoßen, um die Nadeln (4) in die Farbe (2) einzutauchen. Diese Situation ist in der FIG. 2 dargestellt.

Ein Tätowier- oder PMU-Stift besitzt eine oder mehrere parallele, mit geringem Abstand zueinander angeordnete Nadeln (4). Im Falle einer Nadel wird diese infolge von Adhäsionskräften benetzt; im Falle mehrerer Nadeln füllen sich die Zwischenräume anhand der Kapillarwirkung und Adhäsionskraft teilweise mit der Farbe. Anschließend werden die benetzten Nadeln (4) mit den mit Farbe gefüllten Zwischenräumen an der entsprechenden Stelle des Körpers eines Kunden in die Haut hineingestochen, um die Farbe dort zu deponieren.

Der Farbbehälter wird entsorgt, wenn die Behandlung eines Kunden beendet ist oder wenn der Farbbehälter entleert ist.

Die mit der vorliegenden Erfindung erzielten Vorteile bestehen insbesondere darin, dass keine Farbe aus Vorratsflaschen zum Tätowieren abgefüllt werden muss. Dies führt zur Ersparnis von Vorratsflaschen und zu einer Minimierung der Gefahr einer Verkeimung, Infizierung, Verschmutzung und Austrocknung der Farben.

Bei dem erfindungsgemäßen Farbbehälter und dem erfindungsgemäßen Verfahren wird jeder unnötige Kontakt beim Abfüllen, Öffnen oder Ausdrücken von Farbbehältern mit den Händen vermieden.

## Patentansprüche

1. Farbbehälter für Tattoo- oder Permanent-Make-up-Farbe, welcher aus einem Topf (1) besteht, der eine für mindestens eine, vorzugsweise genau eine Tattoo- oder Permanent-Make-up-Behandlung erforderliche Menge an Tattoo- oder Permanent-Make-up-Farbe (2) enthält,
**dadurch gekennzeichnet,**
**dass** der Topf (1) mit einer von Tätowiernadeln (4) eines Tattoo- oder Permanent-Make-up-Stiftes (5) durchdringbaren Folie (3) dicht verschlossen ist, wobei die Folie (3) aus einem bioverträglichen und dünneren Kunststoff besteht als der Topf (1).

2. Farbbehälter nach Anspruch 1,
**dadurch gekennzeichnet,**
**dass** die Folie (3) mit dem Topf (1) verschweißt oder verklebt ist.

3. Farbbehälter nach Anspruch 1 oder 2,
**dadurch gekennzeichnet,**
**dass** der zu einer offenen Seite hin gerichtete Abschluss des Topfes (1) als Wulst ausgebildet ist.

4. Farbbehälter für Tattoo- oder Permanent-Make-up-Farbe nach einem der vorangegangenen Ansprüche,
**dadurch gekennzeichnet,**
**dass** der Topf (1) einen Zylinder mit Ausnahme einer Grundfläche formbündig umschließt.

5. Farbbehälter für Tattoo- oder Permanent-Make-up-Farbe nach einem der vorangegangenen Ansprüche,
**dadurch gekennzeichnet,**
**dass** der Topf (1) aus einem bioverträglichen Material besteht.

6. Farbbehälter für Tattoo- oder Permanent-Make-up-Farbe nach einem oder mehreren der vorangegangenen Ansprüche,
**dadurch gekennzeichnet,**
**dass** die den Topf (1) verschließende Folie (3) aus einem bioverträglichen Material besteht.

7. Farbbehälter für Tattoo- oder Permanent-Make-up-Farbe nach einem oder mehreren der vorangegangenen Ansprüche,
**dadurch gekennzeichnet,**
**dass** er unter sterilen Bedingungen in einer Umverpackung verpackt wird und bis zur Benutzung in dieser Verpackung aufbewahrt wird.

8. Farbbehälter für Tattoo- oder Permanent-Make-up-Farbe nach einem oder mehreren der vorangegangenen Ansprüche,
**dadurch gekennzeichnet,**
**dass** er nach einem Verpacken in eine Umverpackung durch eine Behandlung mit γ-Strahlung sterilisiert wird und bis zur Benutzung in der Umverpackung aufbewahrt wird.

9. Verfahren zum Aufbringen von Tattoos oder Permanent-Make-up auf einen menschlichen oder tierischen Körper mit Hilfe von Nadelnunter Verwendung eines Farbbehälters nach einem der vorangegangenen Ansprüchen,
**dadurch gekennzeichnet,**
**dass** eine einen Farbbehälter verschließende Folie (3) mit der Nadeln (4) durchstoßen wird, die Nadeln (4) in eine in dem Farbbehälter befindliche Farbe (2) eingetaucht werden und die Nadeln (4) nachfolgend in die Haut des menschlichen oder tierischen Körpers hineingestochen werden, um die Farbe (2) dort zu deponieren.

## Claims

1. A ink container for tattoo ink or permanent make-up ink, consisting of a pot (1) containing a quantity of tattoo ink or permanent make-up ink (2) that is sufficient for at least one, preferably precisely one, tattoo ink or permanent make-up treatment,
**characterized in that**
the pot (1) is tightly sealed by a film (3) that can be punctured by tattooing needles (4) of a tattooing pen or permanent make-up pen (5), whereby the film (3) is made of a biocompatible plastic that is thinner than the plastic of the pot (1).

2. The ink container according to claim 1,
**characterized in that**
the film (3) is fused or glued to the pot (1).

3. The ink container according to claim 1 or 2,
**characterized in that**
the closure of the pot (1) facing the open side is configured as a bead.

4. The ink container for tattoo ink or permanent make-up ink according to one of the preceding claims,
**characterized in that**
the pot (1) encloses a cylinder in a form-fitting manner, except for the bottom surface.

5. The ink container for tattoo ink or permanent make-up ink according to one of the preceding claims,
**characterized in that**,
the pot (1) is made of a biocompatible material.

6. The ink container for tattoo ink or permanent make-up ink according to one or more of the preceding claims,
**characterized in that**
the film (3) that closes the pot (1) is made of a biocompatible material.

7. The ink container for tattoo ink or permanent make-up ink according to one or more of the preceding claims,
**characterized in that**
it is packaged in outer packaging under sterile conditions and is stored in this packaging until it is used.

8. The ink container for tattoo ink or permanent make-up ink according to one or more of the preceding claims,
**characterized in that**,
after being packaged in outer packaging, it is sterilized by a treatment with gamma radiation and stored in the outer packaging until it is used.

9. A method for applying tattoos or permanent make-up on a human or animal body by means of needles, using an ink container according to one of the preceding claims,
**characterized in that**
a film (3) that seals an ink container is punctured with the needles (4), the needles (4) are dipped into an ink (2) held in the ink container, and the needles (4) are subsequently inserted into the skin of the human or animal body in order to deposit the ink (2) there.

## Revendications

1. Récipient pour couleur de tatouage ou de maquillage permanent, composé d'un pot (1) qui contient une quantité de couleur de tatouage ou de maquillage permanent requise pour au moins un, préférentiellement exactement un traitement de tatouage ou de maquillage permanent, **caractérisé en ce que** le pot (1) est fermé de manière étanche par une feuille (3) susceptible d'être traversée par des aiguilles de tatouage (4) d'un stylo de tatouage ou de maquillage permanent (5), la feuille (3) étant composée d'une matière plastique biocompatible plus mince que le pot (1).

2. Récipient pour couleur selon la revendication 1, **caractérisé en ce que** la feuille (3) est soudée ou collée sur le pot (1).

3. Récipient pour couleur selon la revendication 1 ou 2, **caractérisé en ce que** la terminaison du pot (1) tournée vers un côté ouvert se présente sous la forme d'un rebord.

4. Récipient pour couleur de tatouage ou de maquillage permanent selon l'une des revendications précédentes, **caractérisé en ce que** le pot (1) entoure par complémentarité de forme un cylindre à l'exception d'une surface de base.

5. Récipient pour couleur de tatouage ou de maquillage permanent selon l'une des revendications précédentes, **caractérisé en ce que** le pot (1) se compose d'un matériau biocompatible.

6. Récipient pour couleur de tatouage ou de maquillage permanent selon l'une ou plusieurs des revendications précédentes, **caractérisé en ce que** la feuille (3) qui ferme le pot (1) se compose d'un matériau biocompatible.

7. Récipient pour couleur de tatouage ou de maquillage permanent selon l'une ou plusieurs des revendications précédentes, **caractérisé en ce qu'**il est emballé dans un suremballage dans des conditions stériles et est conservé dans cet emballage jusqu'à ce qu'il soit utilisé.

8. Récipient pour couleur de tatouage ou de maquillage permanent selon l'une ou plusieurs des revendications précédentes, **caractérisé en ce qu'**il est stérilisé par un rayonnement y après avoir été emballé dans un suremballage et est conservé dans le suremballage jusqu'à ce qu'il soit utilisé.

9. Procédé pour appliquer des tatouages ou un maquillage permanent sur un corps humain ou animal à l'aide d'aiguilles en utilisant un récipient pour couleur selon l'une des revendications précédentes, **caractérisé en ce qu'**une feuille (3) fermant un récipient pour couleur est percée par les aiguilles (4), **en ce que** les aiguilles (4) sont immergées dans une couleur (2) qui se trouve dans le récipient pour couleur et **en ce que** les aiguilles (4) sont ensuite piquées dans la peau du corps humain ou animal pour y déposer la couleur (2).
